# EUROPEAN PATENT SPECIFICATION

(11) **EP 2 976 119 B1**
(45) Date of publication and mention of the grant of the patent: **21.06.2017**
(21) Application number: 14723879.4
(22) Date of filing: 24.03.2014
(51) Int. Cl.: A61M 11/04, A61M 11/06, A61M 16/10, A61M 16/00, A61M 16/06, A61M 16/14, A61M 16/20

(54) **NEBULIZER DEVICE AND MASK OF INHALATION SOLUTION**
VERNEBLERVORRICHTUNG UND MASKE FÜR EINE INHALATIONSLÖSUNG
DISPOSITIF DE NÉBULISATION ET MASQUE POUR SOLUTION D'INHALATION

(30) Priority: 22.03.2013 GR 20130100171
(43) Date of publication of application: 27.01.2016
(73) Proprietor: NAOUM, George, 11475 Gkizi Attikis (GR)
(72) Inventor: NAOUM, George, 11475 Gkizi Attikis (GR)
(74) Representative: ZBM Patents - Zea, Barlocci & Markvardsen
(86) International application number: PCT/GR2014/000019
(87) International publication number: WO 2014/147430

(56) References cited:
- EP-A1- 1 369 141
- WO-A1-2012/085740
- AU-B2- 2002 301 057
- US-A- 3 045 670
- US-A- 4 190 046
- US-A- 4 195 044
- US-A- 4 911 157
- US-A- 5 259 370
- US-A- 5 727 542
- US-A1- 2003 120 157
- US-A1- 2008 142 010
- US-A1- 2008 271 732
- US-B2- 7 396 995

## Description

The invention relates to a system of hot-nebulized solution provision, consisted of an air pipe - duct with embodied system of connectors - power connections - cables, in a device of nebulizer, for the controlled provision of heated nebulized pharmaceutical solution or hot nebulized solution containing essential oils and decoctions' distillates or simple hot nebula for inhalation, and a mask for the therapy or prevention of diseases of the respiratory system. Its use is suggested either for hospital use connected with the central oxygen provider or for therapy at home connected with a device providing compressed air.

There are many types of devices and masks providing inhaled pharmaceutical solutions either in hospitalization or at home nursing. In the first case the devices function by their connection with the central oxygen provider and in the second case with the connection with a portable a device providing compressed air.

In both practices the temperature of the pharmaceutical solution or the normal saline with any additions is low, about 10 Celsius degrees and does not have the therapeutic advantage of hot steam, which is a very important disadvantage in the everyday medical practice. Attempts to provide effective heating of the liquid to be nebulized are illustrated in US4190046 A. Attempts to heat an aerosol before it is administrated to the patient are illustrated in US4911157 A and US2008/0271732 A1. The present application has important advantages against the already existing devices, and for their understanding the reference in some parameters is required.

From the traditions of most of the people we know that in order to assist the organism to confront infections and disorders of the respiratory system, is proposed besides everything else, the consumption of hot decoctions or drinks and the use of humidifiers in the rooms (mainly in children's rooms).

This is justified because the people and the scientific medical knowledge of fluctuant level, through the crossing of centuries, noticed that various drinks and hot water vapors offer warmth in the mucosa and accelerate the therapy. By humidifying with hot steam the mucosa of the bronchial tree, the excreta are liquefied and discharged easier, the surface of the mucosa is cleaned and so is succeeded the action of the pharmaceutical substance transferred by the steam and the warmth of hot steam and medicinal characteristics of the decoctions in the cells of the mucosa of the bronchial tree.

This is also supported by modern medical practice worldwide. In addition, today science knows that hot steam, especially hot nebulized solutions act profitably in human organism.

The benefits from the inhalation of hot nebulized solutions in mild infections of the respiratory system are pointed out in the internet, for example at the NHS for the public (NHS choices), as well as in heavy infections of the respiratory system as mentioned in the international literature, by the use of moisture and heat of the inhaled gas in body temperature in Intensive Care Unit conditions (Crit Care Med 1996; 24:1920 - 1929).

This brief description of the benefits of inhaled hot steam in the bronchial tree, 2, 06 leads to the view that it is more profitable to add the affection of heat, in various therapies with inhaled bronchodilators, anti-inflammatory and antibiotics applied till today in every day medical practice.

By the nebulizers known today, the temperature of the inhaled final product varies between 10 to 15 °C, respectively to the volume and the speed of the flow of the air provided by the devices. The cold nebulized solution with or without added medicines, reaches the mucosa of the respiratory system that has temperature of 37 °C up to 40 °C in infections.

Despite the heat that gets by the air ducts of the upper respiratory system (cavity of the mouth, oral part of pharynx, larynx), the temperature of the nebula that reaches the lower respiratory system is much lower than the temperature of the mucosa, and that does not offer the required warmth in the respiratory system.

In addition by the gases kinetics we know that, the duplication of the temperature increases the speed of masses of gasses per 1.41 times in stable conditions of pressure, volume and composition.

That means that the nebulized **solution of the medicinal substance** of 40°C will have a transfer speed 1.41 times greater than the speed of the steam of 20°C serum.

Keeping in view that by the nebulizers being used the temperature of the inhaled final nebula varies between 10 to 15 °C is concluded that the speed of transfer of the spay of 35 to 40 °C will be almost triple.

By all odds in this case by inhalation the spray will reach in a larger depth in network of air ducts of the lung and therefore the therapeutic result will be better and the cure will be quicker.

The serum constitutes the excipient of the added chemical substance of the bronchodilators or anti-inflammatory medicines or antibiotics or oils, which by its spinning in the device is nebulized-The nebulized solution in 40°C has lower density per cc and more than double speed of transfer from the one in 20°C. These characteristic of the hot nebula, enables it to be easier inhaled and as a result to enter in a greater depth of the bronchial tree in the unit of the time and with a deeper inhalation to reach up to the level of small air ducts.

It is obvious that in case of inhalation of hot nebulas of the therapeutic substance, the therapeutic result will be better. In addition we will have the profitable effect of the heat in the anaplasis of the sick mucosa of the bronchial tree and the best liquidation and absorption of excretions.

For the above mentioned reasons we have a greater bronchodilitation, assistance, the therapy of mucosa, the facilitation of abortion of the excretions via the expectoration, the amelioration of the respiratory ability of the patient, and its quicker.

The same happens in home nursing too, by the use of the nebulizer.

Beside the therapy of the diseases of the respiratory system important value has the preventive cleaning of the respiratory system.

The inhaled air of the encumbered environment of the modern society, mainly of the large urban centers, causes permanent troubles in the normal function of the mucosa of the respiratory system.

The respiratory system of smokers and employees in close work areas with technical heat - freezing, half-freshened air and industries, mines etc, is aggravated more.

Creeping small infections, increase of the production of high viscosity excretions, difficulty in hawking and cleaning of the bronchial tree, constitute some of the encumbrances of the respiratory operation. The common and most of times permanent aggravated of the operation of the respiratory system increases the possibility of pathologic troubles of the respiratory system such as chronic bronchitis, bronchial asthma, COPD (chronic obstructive pulmonary disease, emphysema, lung cancer etc.

The application with the device of frequent cleanings of the respiratory system, with weekly doses of inhalations of heat nebula and the addition of essential oils of eucalyptus or decoctions, constitutes a choice of **preventive therapy** of the respiratory system in order to preserve the organism from pathologic troubles and to guaranty to human being a better quality of life.
In addition is known that by the total of the provided therapeutic substance through inhaled medicines, only 10% approximately reaches the pneumonic parenchyma which is the target for treatment.

It happens because we have losses during inhalation and expiration, as follows:
- During expiration the produced spray is not inhaled, as it is logic, neither is aborted in the environment, so we have an important loss.
- During inhalation and respectively to the inhaling ability of each patient, 10% to 30% of the produced spray is not inhaled.
- The particles of the pharmaceutical substance of 2-5 microns are subject to precipitation and infraction in the mucosa of air ducts of the respiratory system, whereas the particles being smaller than 2 microns are expired.
- 30% to 60% of the medicine lays down in the oral part of the pharynx during the inhalation.
- Approximately 10% stay in the walls of the device.
Considering the scientific marking we proceed to the study and development of a nebulizer device and an inhalation mask with an embodied system for simultaneous heating of the normal saline or the normal saline mixed with essential oils or the pharmaceutical solution and of the produced nebula, with temperature regulator of 35-40°C which is pleasurably affordable by the patient, easy to use and absolutely safe. The invention is defined by the appended claim 1. In an example, the device consists of
1) The air - power pipe-conductor
2) The device of nebulization of the serum (nebulizer) and
3) The inhalation mask

### More specifically:

### Air - power pipe - conductor:

In the two ends of the pipe - conductor (1) there are embodied, a thin safety **power connection** (2) in the central end and a safety **electric point** (3) in the peripheral end, being connected with thin cables (4) across the body of the treatment pipe. The central end ends at the supply of the air volume, which supply bears a thin safety **electric point** (5). The same stands, on the opposite direction in the peripheral end where the electric point (3) of the treatment pipe supplies power to the power connection (6) located in the base of the nebulization device (16), peripherically to the reception mouth of the treatment pipe. By this construction we simultaneously have supply of air and power without complicating or obstructing the common movement of connection of the air duct with the supply, as it is currently applied worldwide.

### Device of nebulization of the serum (nebulizer):

The nebulizer (16) is consisted of the main body of spraying (7) with conical cuplike internal base. The interior of the device is coated with a thin and slim leaf of refractory material (8) that embodies a rich network of heat resistances (9) in its whole internal surface connected with the power connection (6). The coating heats simultaneously the included serum or solution and the produced for inhalation nebula, in the regulated by the regulator (10) temperature up to 40°C.
The upper outlet of the spray (11) is round, and in the back side it bears a conical flat area (12). In the anterior edge of the mouth there is an electronic sensor (17), sensitive in the fluctuation of the pressure of the air by the expiration of the patient. By this stimulant of the patient has the ability to change the polarity of a semi-circular electromagnet (13) in the surface of the edge of the mouth or electromagnets (18), located in the internal surface of the spray exit mouth. In the surface of the flat area is based a circular silicone leaf (14) of diameter equal to the mouth, which moves upwards and downwards, closes or opens the equal in size mouth of the nebula's exit, excludes or permits respectively the exit of the air to the mask of inhalation and brings to periphery magnet poles (15) of opposite polarity (heteronyms) with the electromagnet (13) of the mouth of the edge of the exit that results to the closing of the mouth. In the other version the sensor (17) changes the polarity of the electromagnets (18) and by a proportional way (homonyms to heteronyms charges): a) moves around its axis by vertical to horizontal position and vice versa, a round impeller (19), of equal diameter with the nozzle of the mouth that in periphery has a couple of magnets (20) or b) two wings of semi-circular shape move, of total diameter equal to the cross-section of the mouth, based on the walls of the nozzle (22), move from horizontal position to vertical and vice versa and obstacle or permit the passage of the spray. In the end of each expiration and before the beginning of the inhalation, the sensor (17) changes the polarity of the magnets (13) or (18) and the leaf of silicone or the impellers (19) or (21) close the mouth (11), and obstacle the exit of the steam in order to save the pharmaceutical substance, which by the devices being used is lost in the environment. In addition, whereas the mouth is close, inside the device the pressure of the steam is increased and therefore in the phase of inhalation, the steam is ejected with greater pressure, density and temperature towards the mouth and the nose of the patient, with obvious better results.

By another version, the sensor (17) activates a thin mechanical system consisted of springs found at the same place (13) or (18) and the specific system has exactly the same valvular operation with electromagnets.

### Inhalation Mask:

An inhalation mask with the suitable constructions of conical channels at the internal side, routes the expired air towards the mouth of exit of the nebula, where the sensor is found and vice versa during inhalation, the nebula to the mouth and nasal cavity.
This invention differs and excels from other inventions having the same purpose, namely the provisions of heat solutions for inhalation. For example, in comparison with the U/S patent no 4,911,157/27-03-1990 or the US2008142010, or US3045670 the present application differs among others in the following:
1) it has an embodied to the air pipe - air-duct system, an electric point
2) simultaneously heats the solution and the nebula at the temperature of choice
3) has a system of periodic interruption and provision of spray during expiration and inhalation respectively.
4) Is a simple device, easy to use, safe, light and effective
5) the required actions for its installation are very simple in comparison with the common nebulizer devices.
6) it is a nebulizer, not humidifier and it produces nebula of droplets of liquid and pharmaceutical substance from the high pressure gas according to the Bernulli law.
7) the interior of the whole device is coated with a refractory material
8)) it comprises a rich network of heat resistances (9) in its whole internal surface with which it heats simultaneously the solution and the produced nebula, so that temperature of choice is always the same for the nebula until the inhalation outlet

The drawings that follow describe the device for the best understanding of the reader:

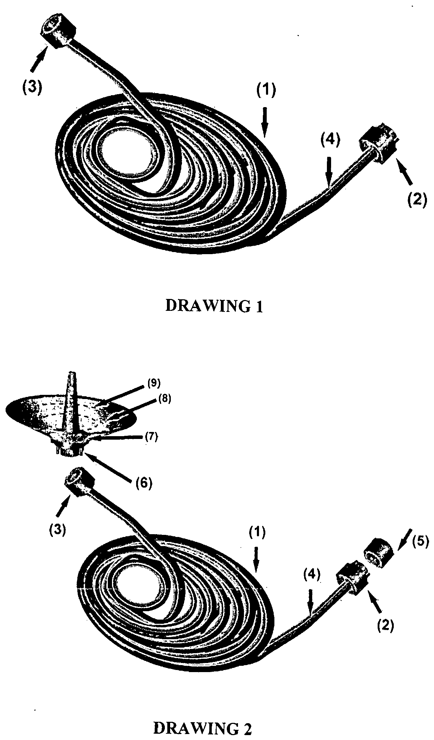

**Drawing 1****:** The air-power pipe - conductor with the two (2) edges, central and peripheral
**Drawing 2****:** The air-power pipe - conductor with the corresponding connections of the edges with the electric point (central power and air supply) and power connection (nebulizer device)

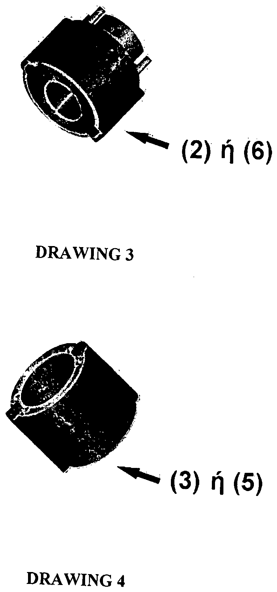

**Drawing 3 and 4****:** Description of the electric points and power connections

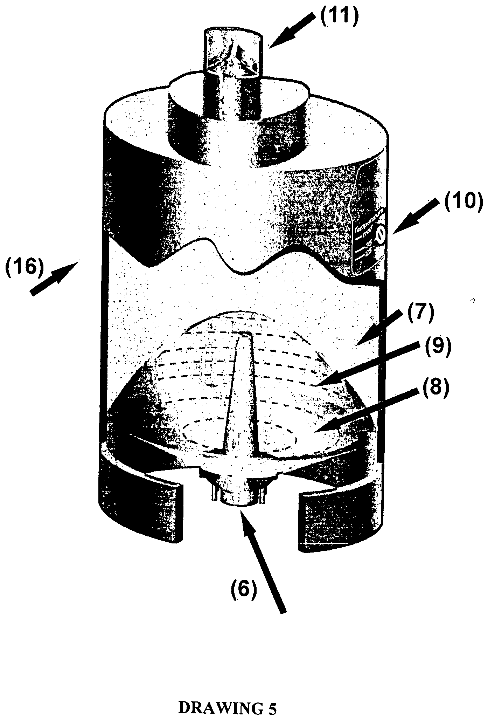

**Drawing 5****:** The nebulizer device

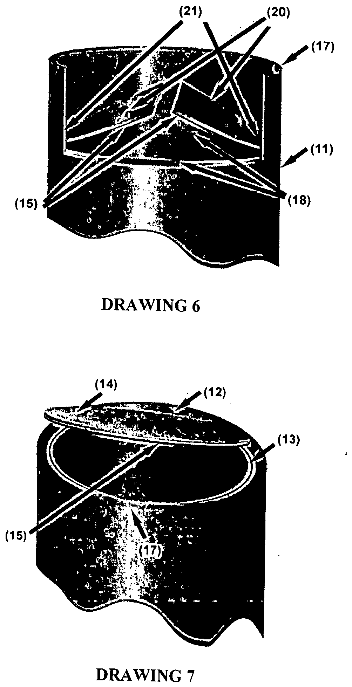

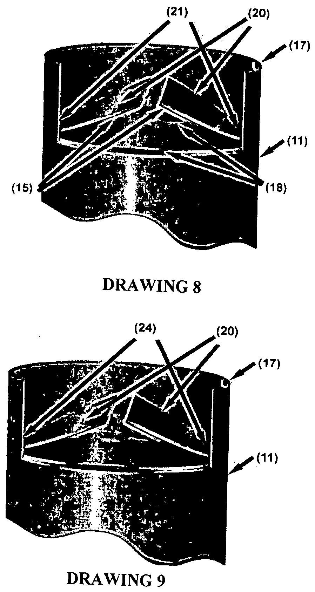

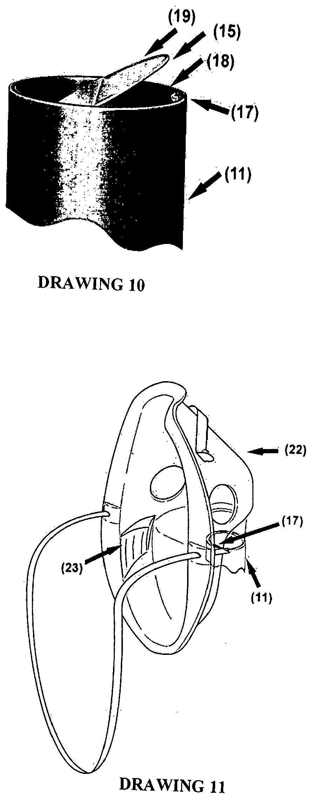

**Drawing 6, 7****,** **8, 9****,** **10****:** The alternative systems (valves) for the controlled provision of spray
**Drawing 11****:** Inhalation Mask

## Claims

1. A nebulizer device (16), connectable to an inhalation mask (22), the nebulizer device comprising:
a main body, including:
a solution;
an interior (7), coated with a coating of refractory material (8) the entire coating incorporating a rich network of heat resistances (9);
a reception mouth (6), connectable to an air-power pipe conductor (1), to receive air and power from the air-power pipe conductor (1) to supply power to the heat resistances and supply air to the interior to produce a nebula from the included solution;
an outlet (11) to provide the produced nebula to the inhalation mask;
a system of periodic interruption and provision of the nebula during expiration and inhalation, respectively,
wherein the heat resistances are configured to heat simultaneously the solution and the produced nebula.

2. The nebulizer device (16) according to claim 1, further comprising an air-power pipe conductor (1), wherein the air-power pipe conductor (1) has a body and two ends, wherein one end of the two ends comprises a safety power connection (2) and the other end of the two ends comprises a safety electric point (3), said two ends being connected with thin cables (4) across the body of the air-power pipe conductor, the one end is connectable to an air volume supply, the air volume supply bearing a safety electric point (5); the safety electric point (3) of the air power pipe conductor provides power to a power connection located at the reception mouth (6) located at the base of the interior (7), peripherically to the reception mouth.

3. The nebulizer device according to claim 2, wherein:
the base is a conical cuplike internal base, said heat resistances are connected to the power connection of the reception mouth (6), and wherein,
the nebulizer device (16) further comprises a temperature regulator (10) to regulate the temperature of the solution and the nebula between 35°C- 40°C;
the outlet (11) is round and bears at a back side a conical flat area (12) of diameter equal to the outlet mouth's diameter ;
in a surface of an internal edge of the mouth of the outlet there are semi-circular electromagnets (13);
wherein the system of periodic interruption and provision of the nebula during expiration and inhalation comprises:
an electronic sensor (17), in an anterior edge of the mouth of the outlet (11), sensitive in the fluctuation of the pressure of the air by the expiration of the patient, and
a circular silicone leaf (14) of diameter equal to the mouth's diameter is based in a surface of the conical flat area (12), said circular silicone leaf being moveable upwards and downwards
the silicone leaf (14) has in its periphery magnetic poles (15);
wherein the sensor is configured to change the polarity of the the electromagnets (13) to move the silicone leaf and close the outlet (11) when the patient expires.

4. The nebulizer device according to claim 2, wherein:
the base is a conical cuplike internal base, said heat resistances are connected to the power connection of the reception mouth (6), and wherein,
the nebulizer device (16) further comprises a temperature regulator (10) to regulate the temperature of the solution and the nebula between 35oC-40oC;
the outlet (11) is round and bears at a back side a conical flat area (12) of diameter equal to the outlet mouth's diameter and is configured to open or close;
in a surface of an internal edge of the mouth of the outlet there are semi-circular electromagnets (13);
wherein the system of periodic interruption and provision of the nebula during expiration and inhalation comprises:
an electronic sensor (17), in an anterior edge of the mouth of the outlet (11), sensitive in the fluctuation of the pressure of the air by the expiration of the patient, and
a round impeller (19) of diameter equal to the diameter of the mouth having a pair of magnets (20) at its periphery, the round impeller (19) is configured to move from vertical to horizontal position and vice versa and
wherein the sensor is configured to change the polarity of the electromagnets (13) to be of opposite polarity to the polarity of the pair of magnets (20) when the patient expires to move the round impeller from vertical to horizontal position to close the outlet (11).

5. The nebulizer device according to claim 2, wherein:
the base is a conical cuplike internal base, said heat resistances are connected to the power connection of the reception mouth (6), and wherein,
the nebulizer device (16) further comprises a temperature regulator (10) to regulate the temperature of the solution and the nebula between 35oC- 40oC;
the outlet (11) is round and bears at a back side a conical flat area (12) of diameter equal to the outlet mouth's diameter and is configured to open or close;
in a surface of an internal edge of the mouth of the outlet there are semi-circular electromagnets (13);
wherein the system of periodic interruption and provision of the nebula during expiration and inhalation comprises:
an electronic sensor (17), in an anterior edge of the mouth of the outlet (11), sensitive in the fluctuation of the pressure of the air by the expiration of the patient, and
two wings (20) of semi-circular shape based on walls (21) of the mouth of the outlet (11) and of total diameter equal to the cross-section of the mouth of the outlet (11), and configured to move from horizontal position to vertical and vice versa,
wherein the sensor is configured to change the polarity of the electromagnets (13) to move the wings from horizontal position to vertical and vice versa to stop or permit the passage of the nebula, respectively.

6. The nebulizer device according to claim 2, wherein:
at a surface of an internal edge of the outlet (11) there is a thin mechanical system (24) consisting of thin springs; a back side bears a conical flat area (12) of diameter equal to the mouth's diameter, coupled to the outlet; in an anterior edge of the outlet (11) there is an electronic sensor (17), sensitive to fluctuations of the pressure of the air by the expiration of the patient and configured to activate the thin mechanical system (24) in response to the sensing to interrupt and provide the nebula during expiration and inhalation, respectively.

7. The nebulizer device according to any of claims 3 to 6, further comprising an inhalation mask (22), wherein the inhalation mask (22) has constructions of conical channels (23) at the internal side configured to route the expired air towards the mouth of the outlet (11), where the electronic sensor (17) is found, and to route the nebula to the mouth and nasal cavity of the patient.

8. The nebulizer device according to any of claims 1-7, wherein the solution is saline.

9. The nebulizer device according to any of claims 1-7, wherein the solution is saline mixed with essential oils.

10. The nebulizer device according to any of claims 1-7, wherein the solution is saline mixed with decoctions' distillates.

11. The nebulizer device according to any of claims 1-7, where the solution includes a pharmaceutical substance.

## Patentansprüche

1. Eine an eine Inhalationsmaske (22) anschließbare Verneblungsvorrichtung (16), wobei die Verneblungsvorrichtung folgendes umfasst:
einen Hauptkörper, umfassend:
eine Lösung;
einen mit einer Beschichtung aus Feuerfestwerkstoff (8) beschichteten Innenbereich (7), wobei die gesamte Beschichtung ein reichhaltiges Netzwerk von Wärmewiderständen (9) einbezieht;
eine an eine Luft-Strom-Rohrleitung (1) anschließbare Empfangsöffnung (6), um Luft und Strom aus der Luft-Strom-Rohrleitung (1) zu empfangen, um die Wärmewiderstände mit Strom zu versorgen und den Innenbereich mit Luft zu versorgen, um einen Nebel aus der enthaltenen Lösung zu erzeugen;
einen Auslass (11), um die Inhalationsmaske mit dem erzeugten Nebel zu versorgen;
ein System periodischer Unterbrechung und Versorgung des Nebels jeweils während der Aus- und Einatmung,
wobei die Wärmewiderstände konfiguriert sind, um gleichzeitig die Lösung und den erzeugten Nebel zu erwärmen.

2. Die Verneblungsvorrichtung (16) nach Anspruch 1, weiterhin umfassend einen Luft-Strom-Rohrleitung (1), wobei die Luft-Strom-Rohrleitung (1) einen Körper und zwei Endbereiche hat, wobei einer der zwei Endbereiche einen Schutzstromanschluss (2) umfasst und der andere Endbereich eine elektrische Schutzweiche (3) umfasst, wobei die zwei Endbereiche entlang des Körpers der Luft-Strom-Rohrleitung mit dünnen Kabeln (4) verbunden sind, wobei der eine Endbereich an eine Luftvolumenversorgungseinheit anschließbar ist, wobei die Luftvolumenversorgungseinheit eine elektrische Schutzweiche (5) aufweist; die elektrische Schutzweiche (3) der Luft-Strom-Rohrleitung einen Stromanschluss, die peripher an der an der Basis des Innenbereichs (7) angeordneten Empfangsöffnung (6) angeordnet ist, mit Strom versorgt.

3. Die Verneblungsvorrichtung nach Anspruch 2, wobei:
die Basis eine konische napfartige innere Basis ist, wobei die Wärmewiderstände an dem Stromanschluss der Empfangsöffnung (6) verbunden sind, und wobei,
die Verneblungsvorrichtung (16) weiterhin einen Temperaturregler (10) zur Regelung der Temperatur der Lösung und des Nebels bei zwischen 35 °C- 40 °C umfasst;
der Auslass (11) rund ist und an einer Hinterseite einen konischen flachen Bereich (12) hat, dessen Durchmesser den Durchmesser der Auslassöffnung gleicht;
in einer Oberfläche einer inneren Kante der Auslassöffnung halbrunde Elektromagneten (13) vorhanden sind;
wobei das System periodischer Unterbrechung und Versorgung des Nebels während der Aus- und Einatmung folgendes umfasst:
einen an einer vorderen Kante der Öffnung des Auslasses (11) angeordneten elektronischen Sensor (17), der empfindlich gegenüber der Schwankung des Luftdrucks bei der Ausatmung durch den Patienten ist, und
ein rundes Silikonblatt (14), dessen Durchmesser den Durchmesser der Öffnung gleicht, das auf einer Oberfläche des konischen flachen Bereichs (12) fußt, wobei das runde Silikonblatt nach oben und nach unten bewogen werden kann
das Silikonblatt (14) im Umfang angeordnete magnetische Polen (15) hat;
wobei der Sensor konfiguriert ist, um die Polarität der Elektromagneten (13) zu ändern, um das Silikonblatt zu bewegen und den Auslass (11) zu schließen, wenn der Patient ausatmet.

4. Die Verneblungsvorrichtung nach Anspruch 2, wobei:
die Basis eine konische napfartige innere Basis ist, wobei die Wärmewiderstände an dem Stromanschluss der Empfangsöffnung (6) verbunden sind, und wobei,
die Verneblungsvorrichtung (16) weiterhin einen Temperaturregler (10) zur Regelung der Temperatur der Lösung und des Nebels bei zwischen 35 °C- 40 °C umfasst;
der Auslass (11) rund ist und an einer Hinterseite einen konischen flachen Bereich (12) aufweist, dessen Durchmesser den Durchmesser der Auslassöffnung gleicht und konfiguriert ist, um sich zu öffnen und zu schließen;
halbrunde Elektromagneten (13) auf einer Oberfläche einer inneren Kante der Auslassöffnung angeordnet sind;
wobei das System periodischer Unterbrechung und Versorgung des Nebels während der Aus- und Einatmung folgendes umfasst:
einen an einer vorderen Kante der Öffnung des Auslasses (11) angeordneten elektronischen Sensor (17), der empfindlich gegenüber der Schwankung des Luftdrucks bei der Ausatmung durch den Patienten ist, und
ein rundes Antriebsrad (19) mit einem Durchmesser, der den Durchmesser der Öffnung gleicht und der ein Paar von Magneten (20) in seinem Umfang hat, wobei das runde Antriebsrad (19) konfiguriert ist, um von einer vertikalen in eine horizontale Position und umgekehrt versetzt zu werden und
wobei der Sensor konfiguriert ist, um die Polarität der Elektromagneten (13) zu ändern, so dass sie der Polarität des Paares von Magneten (20) entgegengesetzt ist, wenn der Patient ausatmet, um das runde Antriebsrad von der vertikalen in die horizontale Position zu versetzen, um den Auslass (11) zu schließen.

5. Die Verneblungsvorrichtung nach Anspruch 2, wobei:
die Basis eine konische napfartige innere Basis ist, wobei die Wärmewiderstände an dem Stromanschluss der Empfangsöffnung (6) verbunden sind, und wobei,
die Verneblungsvorrichtung (16) weiterhin einen Temperaturregler (10) zur Regelung der Temperatur der Lösung und des Nebels bei zwischen 35 °C- 40 °C umfasst;
der Auslass (11) rund ist und an einer Hinterseite einen konischen flachen Bereich (12) aufweist, dessen Durchmesser den Durchmesser der Auslassöffnung gleicht und konfiguriert ist, um sich zu öffnen und zu schließen;
in einer Oberfläche einer inneren Kante der Auslassöffnung halbrunde Elektromagneten (13) vorhanden sind;
wobei das System periodischer Unterbrechung und Versorgung des Nebels während der Aus- und Einatmung folgendes umfasst:
einen an einer vorderen Kante der Öffnung des Auslasses (11) angeordneten elektronischen Sensor (17), der empfindlich gegenüber der Schwankung des Luftdrucks bei der Ausatmung durch den Patienten ist, und
zwei Flügel (20) halbrunder Form, die auf Wänden (21) der Öffnung des Auslasses (11) fußen und deren gesamten Durchmesser den Querschnitt der Öffnung des Auslasses (11) gleicht, und die konfiguriert sind, um von einer horizontalen in eine vertikale Position und umgekehrt versetzt zu werden,
wobei der Sensor konfiguriert ist, um die Polarität der Elektromagneten (13) zu ändern, um die Flügel von der horizontalen Position in die vertikale Position und umgekehrt zu versetzen, um den Durchgang des Nebels jeweils zu halten bzw. zu ermöglichen.

6. Die Verneblungsvorrichtung nach Anspruch 2, wobei:
ein dünnes mechanisches System (24) auf einer Oberfläche einer inneren Kante des Auslasses (11) vorhanden ist, das aus dünnen Federn besteht; eine Rückseite einen an dem Auslass gekoppelten konischen flachen Bereich (12) aufweist, dessen Durchmesser den Durchmesser der Öffnung gleicht; ein elektronischer Sensor (17) an einer vorderen Kante des Auslasses (11) angeordnet ist, der empfindlich gegenüber Schwankungen des Luftdrucks bei der Ausatmung durch den Patienten ist und konfiguriert zur Anregung des dünnen mechanischen Systems (24) als Antwort auf die Erkennung durch den Sensor ist, um den Nebel während der Aus- und Einatmung jeweils zu unterbrechen bzw. zu versorgen.

7. Die Verneblungsvorrichtung nach einem der Ansprüche 3 bis 6, weiterhin umfassend eine Inhalationsmaske (22), wobei die Inhalationsmaske (22) Konstruktionen konischer Kanäle (23) an der inneren Seite hat, die konfiguriert sind, um die ausgeatmete Luft zu der Öffnung des Auslasses (11), wo sich der elektronische Sensor (17) befindet, zu leiten und den Nebel zu der Mund und zur der Nasenhöhle des Patienten zu leiten.

8. Die Verneblungsvorrichtung nach einem der Ansprüche 1-7, wobei die Lösung isotonische Kochsalzlösung ist.

9. Die Verneblungsvorrichtung nach einem der Ansprüche 1-7, wobei die Lösung isotonische Kochsalzlösung gemischt mit ätherischen Ölen ist.

10. Die Verneblungsvorrichtung nach einem der Ansprüche 1-7, wobei die Lösung isotonische Kochsalzlösung gemischt mit Destillaten aus Abkochungen ist.

11. Die Verneblungsvorrichtung nach einem der Ansprüche 1-7, wobei die Lösung eine pharmazeutische Substanz umfasst.

## Revendications

1. Un dispositif de nébulisation (16) puissant être relié à un masque d'inhalation (22), comprenant le dispositif de nébulisation :
un corps principal, incluant :
une solution ;
un intérieur (7), recouvert avec un revêtement de matériau réfractaire (8), incorporant la totalité du revêtement un riche réseau de résistances thermiques (9) ;
une embouchure de réception (6) puissant être reliée à un tube conducteur d'air et de courant électrique (1), pour recevoir de l'air et de la courant électrique du tube conducteur d'air et de courant électrique (1) pour fournir de la courant électrique aux résistances thermiques et fournir de l'air à l'intérieur afin de produire un brouillard à partir de la solution y contenue ;
un orifice de sortie (11) pour fournir le brouillard produit au masque d'inhalation ;
un système d'interruption et de fourniture périodiques du brouillard pendant l'expiration et l'inhalation, respectivement,
dans lequel les résistances thermiques sont configurées pour échauffer simultanément la solution et le brouillard produit.

2. Le dispositif de nébulisation (16) selon la revendication 1, comprenant en outre un tube conducteur d'air et de courant électrique (1), dans lequel le tube conducteur d'air et de courant électrique (1) a un corps et deux parties de bout, dans lequel une des deux parties de bout comprend un raccordement électrique de sécurité (2) et l'autre partie de bout comprend une pointe électrique de sécurité (3), étant les deux parties de bout connectées avec des câbles fins (4) à travers le corps du tube conducteur d'air et de courant électrique, l'une partie de bout peut être reliée à une source de volume d'air, portant la source de volume d'air une pointe électrique de sécurité (5) ; la pointe électrique de sécurité (3) du tube conducteur d'air et de courant électrique fournit de la courant électrique à un raccordement électrique situé dans l'embouchure de réception (6) située dans la base de l'intérieur (7) de façon périphérique à l'embouchure de réception.

3. Le dispositif de nébulisation selon la revendication 2, dans lequel :
la base est une base intérieure en forme de tasse conique, étant lesdites résistances thermiques reliées au raccordement électrique de l'embouchure de réception (6), et dans lequel,
le dispositif de nébulisation (16) comprend en outre un dispositif de régulation de température (10) pour réguler la température de la solution et du brouillard à une valeur d'entre 35 °C- 40 °C ;
l'orifice de sortie (11) est rond et porte dans un côté arrière une surface plane conique (12) ayant un diamètre égal au diamètre de l'embouchure de l'orifice de sortie ;
des électroaimants semi-circulaires (13) sont disposés dans une surface d'une bordure intérieure de l'embouchure de l'orifice de sortie ;
dans lequel le système d'interruption et de fourniture périodiques du brouillard pendant l'expiration et l'inhalation comprend :
un capteur électronique (17), dans une bordure avant de l'embouchure de l'orifice de sortie (11), qui est sensible à la fluctuation de la pression de l'air pendant l'expiration du patient, et
une feuille de silicone circulaire (14) ayant un diamètre égal au diamètre de l'embouchure est située sur une région de la surface plane conique (12), étant ladite feuille de silicone circulaire déplaçable vers le haut et vers le bas
ayant la feuille de silicone (14) dans sa périphérie des pôles magnétiques (15) ;
dans lequel le capteur est configuré pour changer la polarité des électroaimants (13) afin de déplacer la feuille de silicone et fermer l'orifice de sortie (11) lors de l'expiration du patient.

4. Le dispositif de nébulisation selon la revendication 2, dans lequel :
la base est une base intérieure sous forme de tasse conique, lesdites résistances thermiques sont reliées au raccordement électrique de l'embouchure de réception (6), et dans lequel,
le dispositif de nébulisation (16) comprend en outre un dispositif de régulation de température (10) pour réguler la température de la solution et du brouillard à une valeur d'entre 35 °C- 40 °C ;
l'orifice de sortie (11) est rond et porte dans un côté arrière une surface plane conique (12) ayant un diamètre égal au diamètre de l'embouchure de l'orifice de sortie et est configuré pour s'ouvrir et se fermer ;
des électroaimants semi-circulaires (13) sont disposés dans une surface d'une bordure intérieure de l'embouchure de l'orifice de sortie ;
dans lequel le système d'interruption et de fourniture périodiques du brouillard pendant l'expiration et l'inhalation comprend :
un capteur électronique (17), dans une bordure avant de l'embouchure de l'orifice de sortie (11), qui est sensible à la fluctuation de la pression de l'air pendant l'expiration du patient, et
une roue motrice (19) ayant un diamètre égal au diamètre de l'embouchure ayant une paire d'aimants (20) dans sa périphérie, étant configurée la roue motrice (19) pour se déplacer d'une position verticale jusqu'à une position horizontale et vice versa et
dans lequel le capteur est configuré pour changer la polarité des électroaimants (13) afin qu'elle soit opposée à la polarité de la paire d'aimants (20) lors de l'expiration du patient afin de déplacer la roue motrice de la position verticale à la position horizontale pour fermer l'orifice de sortie (11).

5. Le dispositif de nébulisation selon la revendication 2, dans lequel :
la base est une base intérieure sous forme de tasse conique, lesdites résistances thermiques sont reliées au raccordement électrique de l'embouchure de réception (6), et dans lequel,
le dispositif de nébulisation (16) comprend en outre un dispositif de régulation de température (10) pour réguler la température de la solution et du brouillard à une valeur d'entre 35 °C- 40 °C ;
l'orifice de sortie (11) est rond et porte dans un côté arrière une surface plane conique (12) ayant un diamètre égal au diamètre de l'embouchure de l'orifice de sortie et est configuré pour s'ouvrir et se fermer ;
des électroaimants semi-circulaires (13) sont disposés dans une surface d'une bordure intérieure de l'embouchure de l'orifice de sortie ;
dans lequel le système d'interruption et de fourniture périodiques du brouillard pendant l'expiration et l'inhalation comprend :
un capteur électronique (17), dans une bordure avant de l'embouchure de l'orifice de sortie (11), qui est sensible à la fluctuation de la pression de l'air pendant l'expiration du patient, et
deux ailes (20) ayant une forme semi-circulaire situées sur des parois (21) de l'embouchure de l'orifice de sortie (11) et ayant un diamètre total égal à la section transversale de l'embouchure de l'orifice de sortie (11), et configurées pour se déplacer d'une position horizontale jusqu'à une position verticale et vice versa,
dans lequel le capteur est configuré pour changer la polarité des électroaimants (13) pour déplacer les ailes de la position horizontale à la position verticale et vice versa afin d'arrêter ou de permettre le passage du brouillard, respectivement.

6. Le dispositif de nébulisation selon la revendication 2, dans lequel :
dans une surface d'une bordure intérieure de l'orifice de sortie (11) il y a un système mécanique fin (24) consistant en des ressorts fins ; un côté arrière porte une surface plane conique (12) ayant un diamètre égal au diamètre de l'embouchure, couplé à l'orifice de sortie ; dans une bordure avant de l'orifice de sortie (11) il y a an capteur électronique (17) sensible à des fluctuations de la pression de l'air dues à l'expiration du patient et configuré pour activer le système mécanique fin (24) en réponse à la détection afin d'interrompre et de fournir le brouillard pendant l'expiration et l'inhalation, respectivement.

7. Le dispositif de nébulisation selon l'une quelconque des revendications 3 à 6, comprenant en outre un masque d'inhalation (22), dans lequel le masque d'inhalation (22) a des constructions de canaux coniques (23) dans le côté intérieur configurées pour diriger l'air expiré vers l'embouchure de l'orifice de sortie (11), où le capteur électronique (17) est situé, et pour diriger le brouillard vers la bouche et vers la cavité nasale du patient.

8. Le dispositif de nébulisation selon l'une quelconque des revendications 1-7, dans lequel la solution est de la solution saline.

9. Le dispositif de nébulisation selon l'une quelconque des revendications 1-7, dans lequel la solution est de la solution saline mélangée avec des huiles essentiels.

10. Le dispositif de nébulisation selon l'une quelconque des revendications 1-7, dans lequel la solution est de la solution saline mélangée avec des distillats de décoctions.

11. Le dispositif de nébulisation selon l'une quelconque des revendications 1-7, dans lequel la solution inclut une substance pharmaceutique.
